(19)

Europäisches
Patentamt
European
Patent Office
Office européen
des brevets

(11)     **EP 2 741 224 A1**

(12)     **EUROPEAN PATENT APPLICATION**

(43) Date of publication:
**11.06.2014 Bulletin 2014/24**

(51) Int Cl.:
***G06F 19/00*** *(2011.01)*

(21) Application number: **13193508.2**

(22) Date of filing: **19.11.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **20.11.2012 US 201261728600 P
20.11.2012 US 201261728611 P**

(71) Applicant: **Thermo Finnigan LLC
San Jose, CA 95134 (US)**

(72) Inventors:
• **Grothe, Robert A, Jr.
Mountain View, CA 94040 (US)**
• **Blethrow, Justin
Oakland, CA 94602 (US)**

(74) Representative: **Boult Wade Tennant
Verulam Gardens
70 Gray's Inn Road
London WC1X 8BT (GB)**

(54)     **Methods for generating local mass spectral libraries for interpreting multiplexed mass spectra**

(57)     A method of acquiring and compiling data obtained on a mass spectrometer system, comprises: (a) generating a multiplexed mass spectrum comprising a superposed plurality of product-ion mass spectra comprising a plurality of product-ion types, each product-ion mass spectrum corresponding to fragmentation of a respective precursor-ion type, each precursor-ion type and each product ion type having a respective mass-to-charge (m/z) ratio ; (b) decomposing the multiplexed product-ion mass spectrum so as to recognize relative abundances of previously-observed product-ion mass spectra within the multiplexed product-ion mass spectrum, the decomposing employing a mass-spectral library having a plurality of entries corresponding to respective product ion mass spectra previously-observed on said mass spectrometer system; (c) recognizing an additional contribution to the multiplexed mass spectrum that is neither attributable to random variation nor to any previously-observed product-ion spectrum; and (d) storing at least one new entry in the mass-spectral library relating to the recognized additional contribution.

EP 2 741 224 A1

**Description**

FIELD OF THE INVENTION

The present invention relates to methods of and systems for obtaining and analyzing spectra of ion products generated from one or more precursor ions.

BACKGROUND OF THE INVENTION

Structural elucidation of ionized molecules of complex structure, such as proteins, is often carried out using a tandem mass spectrometer, where a particular precursor ion is selected at the first stage of analysis or in a first mass analyzer, the precursor ions are subjected to fragmentation (e.g., in a collision cell), and the resulting fragment (product) ions are transported for analysis in the second stage or second mass analyzer. The method can be extended to provide fragmentation of a selected fragment, and so on, with analysis of the resulting fragments for each generation. This is typically referred to an $MS^n$ spectrometry, with $n$ indicating the number of steps of mass analysis and the number of generations of ions. Accordingly, $MS^2$ mass analysis (also known as an MS/MS mass analysis) corresponds to two stages of mass analysis with two generations of ions analyzed (precursor and products). A resulting product spectrum exhibits a set of fragmentation peaks (a fragment set) which, in many instances, may be used as a fingerprint to derive structural information relating to the parent peptide or protein.

There is currently a trend towards full-scan MS experiments coupled with "all-ions" fragmentation. Such full-scan approaches utilize high performance time-of-flight (TOF) or electrostatic trap (such as Orbitrap®-type) mass spectrometers - possibly coupled to UHPLC columns - and can facilitate rapid and sensitive detection and/or quantitative screening of analytes. The superior resolving power of the Orbitrap® mass spectrometer (up to 100,000 FWHM) compared to TOF instruments (10,000-20,000) ensures the high mass accuracy required for complex sample analysis.

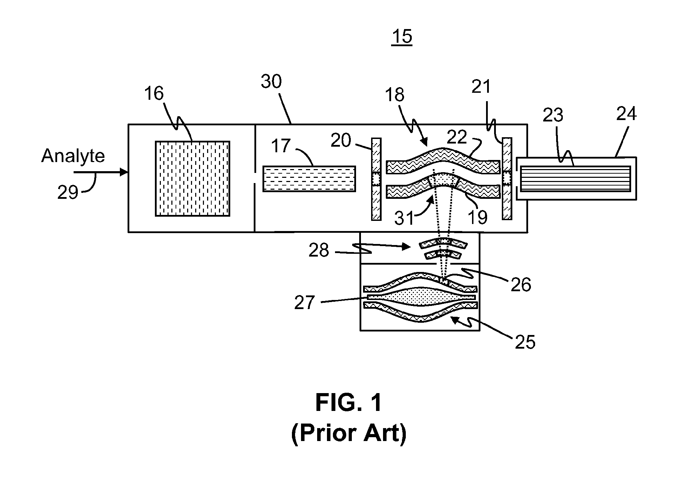

An example of a mass spectrometer system **15** comprising an electrostatic trap mass analyzer such as an Orbitrap® mass analyzer **25** is shown in FIG. 1. Analyte material **29** is provided to a pulsed or continuous ion source **16** so as to generate ions. Ion source **16** could be a MALDI source, an electrospray source or any other type of ion source. In addition, multiple ion sources may be used. The illustrated system comprises a curved quadrupole trap **18** (also known as a "C-trap") with a slot **31** in the inner electrode **19**. Ions are transferred from the ion source **16** to the curved quadrupole trap **18** by ion optics assembly **17** (e.g. an RF multipole). Prior to ion injection, ions may be squeezed along the axis of the curved quadrupole trap **18** by raising voltages on end electrodes **20** and **21.** For ion injection into the Orbitrap® mass analyzer **25,** the RF voltage on the curved quadrupole trap **18** may be switched off, as is well known. Pulses are applied to electrodes **19** and **22** and to an electrode of curved ion optics **28** so that the transverse electric field accelerates ions into the curved ion optics **28**. The converging ion beam that results enters the Orbitrap® mass analyzer **25** through injection slot **26.** The ion beam is squeezed towards the axis by an increasing voltage on a central electrode **27.** Due to temporal and spatial focusing at the injection slot **26,** ions start coherent axial oscillations. These oscillations produce image currents that are amplified and processed. Further details of the electrostatic trap apparatus **25** are described in International Application Publication WO 02/078046, US Pat. No. 5,886,346, US Pat. No. 6,872,938. The ion optics assembly **17,** curved quadrupole trap **18** and associated ion optics are enclosed in a housing **30** which is evacuated in operation of the system.

The system **15** (FIG. 1) further comprises reaction cell **23,** which may comprise a collision cell (such as an octopole) that is enclosed in a gas tight shroud **24** and that is aligned to the curved quadrupole trap **18.** The reaction cell **23,** when used as a collision cell, may be supplied with an RF voltage of which the DC offset can be varied. A collision gas line (not shown) may be attached and the cell is pressurized with nitrogen (or any) gas.

Higher energy collisions (HCD) may take place in the system **15** as follows: Ions are transferred to the curved quadrupole trap **18.** The curved quadrupole trap is held at ground potential. For HCD, ions are emitted from the curved quadrupole trap **18** to the octopole of the reaction cell **23** by setting a voltage on a trap lens. Ions collide with the gas in the reaction cell **23** at an experimentally variable energy which may be represented as a relative energy depending on the ion mass, charge, and also the nature of the collision gas (i.e., a normalized collision energy). Thereafter, the product ions are transferred from the reaction cell back to the curved quadrupole trap by raising the potential of the octopole. A short time delay (for instance 30 ms) is used to ensure that all of the ions are transferred. In the final step, ions are ejected from the curved quadrupole trap **18** into the Orbitrap® mass analyzer **25** as described previously.

The mass spectrometer system **15** illustrated in FIG. 1 lacks a mass filtering step and, instead, causes fragmentation of all precursor ions at once, without first selecting particular precursor ions to fragment. Accordingly, the equivalent of a tandem mass spectrometry experiment is performed as follows: (a) a first sample of ions (comprising a plurality of types of ions) produced from an eluting chemical compound are transferred to and captured by the curved quadrupole trap **18;** (b) the first sample of ions is transferred to the Orbitrap® mass analyzer **25** as described above for analysis, thereby producing a "full-scan" of the ions; (c) after the first sample of ions has been emptied from the curved quadrupole trap **18,** a second sample of ions from the same chemical compound are transferred through the curved quadrupole trap **18** to the reaction cell **23;** (d) in the reaction cell, a plurality of different types of fragment ions are formed from each of the plurality of ion types of the second sample of the chemical compound; (e) once the Orbitrap® mass analyzer **25** has been purged of the first sample of ions, the fragment ions are transferred back quadrupole trap **18** and then to the Orbitrap® analyzer **25** for analysis as described above. Such "all-ions-fragmentation scanning" provides a potential multiplexing advantage, but only if the analysis firmware or software can successfully interpret a collection of detected product ions in terms of a collection of putative precursor ions.

The system **15** shown in FIG. 1 and described above will generally generate $MS^2$ spectral data that may be described as "intentionally multiplexed" because precursor ions are not isolated prior to fragmentation. Conventional triple-quadrupole and other types of mass spectrometer systems may also be employed so as to generate $MS^2$ spectral data and multiplex spectra may be also be generated by these types of systems. However, in these latter cases, any multiplexing may, in fact, be unintentional, since it is often desired, with such instruments, to isolate certain precursor ions of a single *m/z* ratio or ratio range prior to fragmentation. Multiplexing may thus be said to be unintentional when a single isolation window (e.g. 1 Da) happens to contain multiple precursor ions in addition to the one being targeted for selection and fragmentation. The presence of multiplexing could be known or unknown. Regardless of whether multiplexing is intentional or unintentional, or known or unknown, there is a requirement for methods to that can resolve the various multiplexed components.

An $MS^2$ spectrum (a spectrum of fragment or product ions) can provide rich information about the covalent structure of an isolated precursor molecule. The information content is very high in that the $MS^2$ spectrum from one isolated precursor is typically quite different from that of another isolated precursor; furthermore, the $MS^2$ spectrum of a given precursor is highly reproducible. Therefore, in most cases, it is unlikely that (unintentional) experimental and measurement variations in acquiring $MS^2$ spectra would cause one precursor to be mistaken for another. Precursors with similar product ion spectra can often be distinguished by precursor mass or chromatographic retention time. If these additional attributes are insufficient for discrimination, then acquisition of product ion trees (i.e. $MS^3$ and beyond) would be required.

Despite the apparently very high information content of $MS^2$ spectra, the success rate for identifying molecules is very low, ranging from 10-30%. A number of factors may explain the low success rate, including incomplete or poorly curated databases and inadequate software. The spectral decomposition method described herein tolerates an incomplete database and is capable of finding components in a product ion spectrum that exist in the database. A related method of automated database curation adds new entries to the database when it can be determined that the product ion spectrum contains additional components that have not been observed previously.

A more fundamental problem than database quality or completeness is that most software for interpreting product ion spectra begins with the assumption that the observed products are derived from a single isolated precursor. Recent studies have shown that, in typical proteomic studies, the vast majority of product ion spectra are derived from mixtures of precursor ions. Even software packages that address demultiplexing of multiple precursors are closely derived from single-precursor algorithms, with heuristic subtraction-based, computationally-intensive approaches to sequential discovery of multiple precursors. The state-of-the-art in demultiplexing product ion spectra is limited to identification of at most two or three precursors and with very limited abundance dynamic range.

Typical approaches to interpreting product ion spectra consider only the masses of the product ions, and not their relative intensities. Intensity information has been excluded from conventional analyses because it is difficult to predict relative product ion intensities *de novo.* For an approach where the product ion spectra of all potential precursors are assumed to be stored in a library, it is not necessary to predict intensities. Instead, the requirement is that product ion intensities be reproducible. On modern instruments, where acquisition parameters such as collision gas pressure and collision energy are standardized, product ion intensities are highly reproducible. High reproducibility allows quantitatively accurate interpretation of mixed product ion spectra. The inventors have discovered that the product- or fragment-ion intensities provide a very significant amount of information and are quite reproducible on a given instrument using invariant (i.e. standardized) acquisition parameters.

SUMMARY OF THE INVENTION

Disclosed herein is a linear-algebraic approach to analyzing and interpreting multiplex $MS^2$ spectra (fragment- or product-ion spectra) using a spectral library. Also disclosed is a method for automatically constructing a spectral library. In some embodiments, the automatic spectral library construction may be accomplished as an adjunct of the automatic analysis and interpretation process. Specifically, an input spectrum may be decomposed into components of the library, such components comprising previously observed product ion spectra from the library. The residue, or product ions in the observed spectra that cannot be explained in terms of existing library entries, may comprise the basis for adding new entries to the library.

As the term is used herein, a "multiplex" $MS^2$ spectrum contains fragments that arise from a mixture of precursors, in contrast to a "pure" MS$^2$ spectrum in which the fragments come from a single isolated precursor. Further, in the context of this disclosure, "spectral interpretation" means estimating the relative abundance of each precursor represented in the multiplex spectrum. The precursors are assumed to be represented within entries in a database of observed MS$^2$ spectra. Most product ion spectra are interpreted as linear superpositions of library spectra; however, some product ion spectra contain contributions from previously unrecorded precursors that can also be discovered during analysis.

Acquisition of multiplexed product ion spectra may be intentional or unintentional. In some cases, multiple precursors may isolated and combined prior to fragmentation so as to exploit the channel bandwidth of a high-resolution mass-analyzer so as to increase analytical throughput. Multiplexing may be said to be unintentional when a single isolation window (e.g. 1 Da) happens to contains multiple precursor ions in addition to the one being targeted for selection and fragmentation. These additional precursors may be below the limit of detection in an MS$^1$ (precursor ion) spectrum and yet be detectable in MS$^2$ spectra as a consequence of isolation because isolating narrow mass ranges typically involves significantly longer ion accumulation. Alternatively, a single detected peak in an MS$^1$ spectrum may be hiding multiple precursors with mass differences too small to be resolved, or in fact, structural isomers of identical mass. Therefore, the methods described herein make no assumption about the number of precursors. Instead, all candidate precursors within a given mass range are assumed to be present and their intensities (most often zero) are estimated. If, in fact, only one precursor is represented in the MS$^2$ spectrum, the algorithm will work as expected: identifying that precursor by assigning (essentially) zero abundance to all other candidates.

Optimal estimates of precursor abundances are determined from an observed MS$^2$ spectrum by solving a linear matrix equation. Typically, only a few precursor ion candidates are present. If a candidate is not present, its estimated intensity is expected to be near zero. In general, the threshold for discriminating low abundance precursors from precursors that are, in fact, absent, is determined by measurement noise, reproducibility of the database entries, and the similarity among these entries. In various embodiments, the disclosed methods may consider a precursor whose estimated intensity falls below this threshold to be absent, or more precisely, not detected. A candidate that is not represented will have an abundance estimate that is not significantly different from zero. An appropriately chosen threshold is used to eliminate such ions from consideration. In this context, the interpretation can be viewed as precursor ion identification, but with the generalization that it can make multiple simultaneous identifications as well as determining the relative abundances of the precursors. The utility of multiple identification increases dramatically in complex samples, such as proteomic digests.

Accordingly, in one aspect of the present teachings, a method of acquiring and compiling data relating to a plurality of chemical compounds on a mass spectrometer system is disclosed, the method comprising: (a) generating a multiplexed mass spectrum using the mass spectrometer system, the multiplexed mass spectrum comprising a superposition of a plurality of product-ion mass spectra comprising a plurality of product-ion types, each product-ion mass spectrum corresponding to fragmentation of a respective precursor-ion type formed by ionization of the plurality of chemical compounds, each precursor-ion type having a respective precursor-ion mass-to-charge ($m/z$) ratio and each product ion type having a respective product-ion $m/z$ ratio; (b) decomposing the multiplexed product-ion mass spectrum so as to recognize relative abundances of previously-observed product-ion mass spectra within the multiplexed product-ion mass spectrum, the decomposing employing a mass-spectral library having a plurality of entries wherein each entry corresponds to a respective previously-observed product ion mass spectrum, said previous observation being made on said mass spectrometer system; (c) recognizing an additional contribution to the multiplexed product-ion mass spectrum that is neither attributable to random variation nor to any previously-observed product-ion spectrum; and (d) storing at least one new entry in the mass-spectral library relating to the recognized additional contribution..

The step the step (a) of generating a multiplexed mass spectrum using the mass spectrometer system may, in some embodiments, comprise operating the mass spectrometer system according to a rule that specifies instrument operating parameters required for performing the decomposing employing the mass-spectral library. In alternative embodiments, instrument operating parameters may be stored in the mass spectral library.

In some embodiments, the plurality of compounds may elute from a chromatograph at a particular retention time. In such embodiments, a respective profile of detected product-ion intensity versus retention time for each product ion contributing to the recognized additional contribution may be constructed and subsets of the profiles based on correlations between the profiles may be recognized. Subsequently, the additional contribution to the multiplexed product-ion mass spectrum may be decomposed into multiple contributions to the multiplexed product-ion mass spectrum in accordance with the recognized subsets.

In some embodiments, the step (a) comprises fragmenting only precursor-ion types having precursor-ion $m/z$ ratios within a restricted range of $m/z$ ratios. In such cases, the step (b) of decomposing the multiplexed product-ion mass spectrum may comprise employing a segment of the mass spectral library, wherein each entry of the segment of the mass spectral library corresponds to precursor-ion types within the restricted range of $m/z$ ratios.

In various embodiments, the step (d) of storing at least one new entry in the mass-spectral library relating to the recognized additional contribution may comprise: (d1) creating an additional column in a matrix D defined by

$$\mathbf{D} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \begin{bmatrix} d_{n,k} \end{bmatrix}_{N \times K}$$

wherein each element $d_{n,k}$ is an observed intensity of a $k$th product-ion spectrum of the segment of the mass spectral library at an $n$th $m/z$ bin position, $K$ is a total number of product-ion spectra assigned to the library segment and $N$ is a total number of $m/z$ bins defined within the library segment; and (d2) storing, in the mass spectral library, each $\mathbf{D}$ matrix or a respective matrix derived therefrom.

In another aspect, there is disclosed a method of compiling data previously obtained on a mass spectrometer system into a local mass spectral library, comprising: (a) reading a plurality of tandem mass spectra previously obtained using the mass spectrometer system, each tandem mass spectrum comprising detected intensity data for a precursor ion type having a respective precursor-ion mass-to-charge ($m/z$) ratio and an $MS^2$ spectrum comprising detected intensity data for one or more product ion types formed by fragmentation of the precursor ion type and having respective product-ion $m/z$ ratios; (b) sorting the tandem mass spectra according to the precursor-ion $m/z$ ratios; (c) assigning each tandem mass spectrum to one of a plurality of library segments according to its respective precursor-ion $m/z$ ratio, each library segment representing a respective range of precursor-ion $m/z$ ratios; (d) assigning each of the product ion types within each library segment to one of a plurality of bins defined for the library segment, each bin representing a respective range of product-ion $m/z$ ratios; (e) computing, for each library segment, a matrix $\mathbf{D}$ defined by

$$\mathbf{D} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \begin{bmatrix} d_{n,k} \end{bmatrix}_{N \times K}$$

wherein each element $d_{n,k}$ is the observed intensity of the $k$th $MS^2$ spectrum at the $n$th bin position, $K$ is the total number of $MS^2$ spectra assigned to the library segment and $N$ is the total number of $m/z$ bins defined within the library segment; and (f) storing, in the local mass spectral library, each $\mathbf{D}$ matrix or a respective matrix derived therefrom.

In various embodiments, a matrix calculated as $\mathbf{D}^T\mathbf{D}$ may be stored in the local mass spectral library. Its inverse, $(\mathbf{D}^T\mathbf{D})^{-1}$, may be stored in the mass spectral library. Various embodiments may further comprise identifying $MS^2$ spectra in at least one $\mathbf{D}$ matrix using a conventional identification method that recognizes the spectra based on m/z ratios but not detected intensities. In such cases, the entries in the at least one $\mathbf{D}$ matrix may be filtered so as to only include identified $MS^2$ spectra or an annotation relating to at least one identified $MS^2$ spectrum may be stored in the local mass spectral library.

The present teachings assume that a library - possibly an incomplete library - of observed $MS^2$ spectra has been compiled previously. The library may be updated and, in fact, may be constantly updated as new precursors are discovered as an adjunct to the interpretation process. The methods taught herein do not require that all components in the library have been annotated with identifications. When a component identified in a product ion spectrum is matched to a library entry to which an annotation (such as a compound name) is attached, the component is said to be identified. Alternatively, if a component is matched to a library entry to which no annotation is attached, the component is said to be matched. In the case where the library entry is thought to be significant (for example, its abundance discriminates between two patient groups as a putative biomarker), an offline process can be used to identify the entry and to add an annotation.

BRIEF DESCRIPTION OF THE DRAWINGS

The above noted and various other aspects of the present invention will become apparent from the following description which is given by way of example only and with reference to the accompanying drawings, not drawn to scale, in which:

FIG. 1 is a schematic illustration of an example of a mass spectrometer system comprising an electrostatic trap mass analyzer such as an Exactive® mass spectrometer instrument comprising an Orbitrap® mass analyzer and a fragmentation cell but providing no mechanism for isolating precursor ions before fragmentation;

FIG. 2 is a schematic diagram of a system for generating and automatically analyzing chromatography / mass spectrometry spectra in accordance with the present teachings;

FIG. 3 is a schematic illustration of the occurrence of a mixture of precursor ions in an MS$^1$ spectrum, where the presence of multiple precursors may be undetected, giving rise to a multiplexed product ion spectrum;

FIG. 4 is a histogram of spectral library sizes, representing the number of spectral library entries for each nominal precursor ion mass-to-charge ratio, utilized for the test example discussed in the accompanying text;

FIG. 5 is a three-dimensional plot showing estimated intensities for various spectral library entries when each of multiple product ion spectra resulting from isolating the mass-to-charge ratio (*m/z*) range 436-437 are acquired and analyzed, as determined in conjunction with the test example discussed in the accompanying text;

FIG. 6 is a plot showing the spectral library decomposition of three product ion spectra formed by isolating the *m/z* range 709-710, as determined in conjunction with the test example discussed in the accompanying text;

FIG. 7 is a plot of five additional calculated results vectors against the library segment with *m/z* range 709-710 Da, as determined in conjunction with the test example discussed in the accompanying text;

FIG. 8 is a three-dimensional plot of a portion of the search results portrayed as a contour plot, as determined in conjunction with the test example discussed in the accompanying text; and

FIG. 9 is a line plot of the calculated result vectors of various groups of eight non-adjacent test (composite) spectra, as determined in conjunction with the test example discussed in the accompanying text.

DETAILED DESCRIPTION

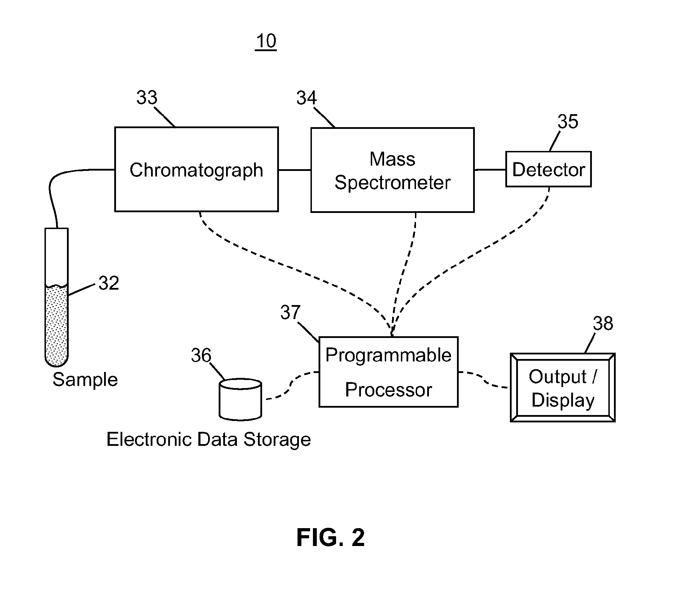

The following description is presented to enable any person skilled in the art to make and use the invention, and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. To fully appreciate the features of the present invention in greater detail, please refer to FIGS. 2-9, in which like reference numbers refer to like elements, in conjunction with the following discussion.

*General Considerations*

FIG. 2 is a schematic diagram of a system for generating and automatically analyzing chromatography / mass spectrometry spectra in accordance with the present teachings. A chromatograph **33,** such as a liquid chromatograph, high-performance liquid chromatograph or ultra high performance liquid chromatograph receives a sample **32** of an analyte mixture and at least partially separates the analyte mixture into individual chemical components, in accordance with well-known chromatographic principles. As a result, the at least partially separated chemical components are transferred to a mass spectrometer **34** at different respective times for mass analysis. As each chemical component is received by the mass spectrometer, it is ionized by an ionization source of the mass spectrometer. The ionization source may produce a plurality of ions (i.e., a plurality of precursor ions) comprising differing charges or masses from each chemical component. Thus, a plurality of ions of differing mass-to-charge ratios may be produced for each chemical component, each such component eluting from the chromatograph at its own characteristic time. These various ions are analyzed and detected by the mass spectrometer together with its detector **35** and, as a result, appropriately identified according to their various mass-to-charge ratios. As illustrated in FIG. 1, the mass spectrometer comprises a reaction cell to fragment or cause other reactions of the precursor ions. In some embodiments, the mass spectrometer may include a mass filtering stage for selection of particular ions to introduce into the reaction cell. However, in other embodiments, the mass spectrometer may lack such a mass filtering stage. In such situations, the reaction cell, instead, causes reactions to or fragmentation of all ions at once.

Still referring to FIG. 2, a programmable processor **37** is electronically coupled to the detector of the mass spectrometer and receives the data produced by the detector during chromatographic / mass spectrometric analysis of the sample(s). The programmable processor may comprise a separate stand-alone computer or may simply comprise a circuit board or any other programmable logic device operated by either firmware or software. Optionally, the programmable processor may also be electronically coupled to the chromatograph and/or the mass spectrometer in order to transmit electronic control signals to one or the other of these instruments so as to control their operation. The nature of such control signals may possibly be determined in response to the data transmitted from the detector to the programmable processor or to the analysis of that data. The programmable processor may also be electronically coupled to a display or other output **38,** for direct output of data or data analysis results to a user, or to electronic data storage **36.**

The programmable processor shown in FIG. 2 is generally operable to: receive a precursor ion chromatography / mass spectrometry spectrum and a product ion chromatography / mass spectrometry spectrum from the chromatography / mass spectrometry apparatus. The programmable processor may be further equipped so as to analyze multiplex $MS^2$ spectral data generated by the mass spectrometer **34.** Alternatively, a second programmable processor or computing system that is not directly coupled to the mass spectrometer may indirectly receive the generated data and perform the data analyses "offline".

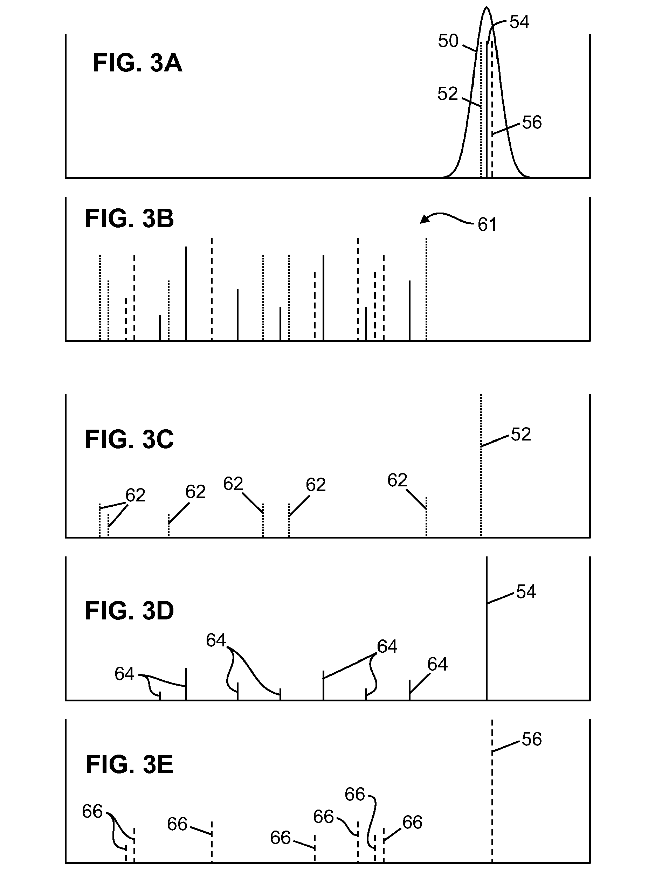

FIG. 3 schematically illustrates a typical problem that may occur during acquisition of a multiplex $MS^2$ spectrum. FIG. 3A illustrates a precursor ion spectrum ($MS^1$ spectrum) that may be obtained within a mass-to-charge envelope or window **50.** The envelope or window may comprise an isolation window or transmission window that is utilized so as to filter an initial plurality of ions such that only ions having mass-to-charge (*m/z*) values encompassed by the window are transmitted to a mass analyzer. Because the envelope or window **50** necessarily comprises a non-zero width, ions comprising more than one *m/z* ratio may be included in the precursor ion spectrum. The inclusion of more than one precursor *m/z* ratio may be either intentional or unintentional as described above.

In the hypothetical example illustrated in FIG. 3A, three different precursor ion types having different *m/z* ratios, represented by the lines **52, 54** and **56,** are included in the population of ions represented by the $MS^1$ spectrum. Simultaneous fragmentation of the precursor ion types produces fragment ions of different respective *m/z* ratios, as are represented by the lines **61** shown in the hypothetical $MS^2$ spectrum of FIG. 3B. Because three different precursor ion types are fragmented, the spectrum illustrated in FIG. 3B is a multiplex $MS^2$ spectrum that comprises first, second and third sub-populations of fragment ions whose various *m/z* ratios are represented, respectively, by dotted lines **62,** solid lines **64** and dashed lines **66.** The problem that is addressed by the present teachings is the recognition of the three different sub-populations of fragment ions and the association of each of the fragment ion sub-populations with a respective precursor ion type. For example, in FIGS. 3C, 3D and 3E, the fragment lines **62** are recognized as a first sub-population and associated with the precursor line **52,** the fragment lines **64** are recognized as a second sub-population and associated with the precursor line **54,** and the fragment lines **66** are recognized as a third sub-population and associated with the precursor line **56,** respectively.

*Mathematical model*

In the novel algorithms described herein, the inventors present a mathematical model for multiplex $MS^2$ spectra, pose a mathematical problem, and offer the solution to that problem. The model for a product ion spectrum resulting from a mixture of precursors is a corresponding mixture of product ion spectra from the isolated precursors. This model has the following linearity property. Let X denote a mixture of precursors A, B, and C in the following proportions: $x_A$ parts of A, $x_B$ parts of B, and c parts of C. We use the following notation to represent this mixture: $X = x_A A + x_B B + x_C C$. Let s(X) denote the "ideal" product ion spectrum obtained from X. In our model, $s(X) = x_A s(A) + x_B s(B) + x_C S(C)$, where s(A), s(B), and s(C) represent "ideal" product ion spectra that would be obtained from isolated (pure) precursors s(A), s(B), and s(C). Each of the "ideal" product ion scans can be thought of as a vector, and so multiplication and addition have their usual intuitive meanings in the above equation.

Now suppose we are presented with a product ion spectrum S. We wish to interpret the product ion spectrum as a mixture of precursors A, B, and C. Then, we suppose that S = s(X), where $X = x_A A + x_B B + x_C C$, i.e. an arbitrary mixture of these precursors. In this case, we do not have ideal product ion spectra s(A), s(B), and s(C). Instead, we assume that we have *measured* product ion spectra of isolated precursors A, B, C, which we will denote by d(A), d(B), d(C). We use the symbol d to suggest that these product ion spectra reside in a database. Given d(A), d(B), and d(C), we can generate arbitrary mixtures of product ion spectra: $S' = x'_A d(A) + x'_B d(B) + x'_C d(C)$, by choosing arbitrary values for coefficients $x'_A$, $x'_B$, and $x'_C$. Specifically, we wish to find coefficients $x'_A$, $x'_B$, and $x'_C$ that minimize the difference between observed product ion spectrum S and our model mixture spectrum S'. Considering S and S' to be vectors, the difference has the geometric interpretation as the length of the difference between two vectors. Geometrically, the set of vectors S' that can be produced from d(A), d(B), and d(C) can be thought of as a hyperplane, where $(x'_A, x'_B, x'_C)$ identify a point in this hyperplane. Let $(\hat{x}_A, \hat{x}_B, \hat{x}_C)$ denote the optimal values of $(x'_A, x'_B, x'_C)$, the coefficient values that minimize the difference between S and S'. According to our model, these optimal coefficients represent the estimates of the coefficients that characterize unknown X that gave rise to product-ion spectrum S as a mixture of precursors A, B, and C. The value of $(\hat{x}_A, \hat{x}_B, \hat{x}_C)$ is simply the projection of S onto the hyperplane determined by vectors d(A), d(B), d(C) and can be calculated by solving a linear matrix equation as shown below.

In general, we are presented with a product ion spectrum derived from a mixture of precursor ions. The mixture of precursor ions may be generated by applying a filter that selects all ions in a given mass range. These ions can be indirectly visualized in a precursor spectrum (e.g., window **50** of FIG. 3A). The precursor spectrum typically covers the entire mass range and thus the ion capacity of the mass analyzer is allocated to the entire spectrum of ions. In the product ion scan, a fragmentation cell is filled only with ions in the selected mass range (isolation window) and these ions are subsequently injected into the mass analyzer. By devoting the entire capacity of the analyzer to this relatively narrow mass range, it is possible to detect ion species in a product ion spectrum that were not detected in the MS$^1$ spectrum. Although a particular precursor of approximately known mass within the isolation window may be specifically "targeted", we will assume that the isolation window may contain any ion whose mass is in the isolation window-whether seen or unseen in the MS$^1$ spectrum. In fact, we will assume that the isolation window includes a linear combination of all masses in the range for which we have a database entry. Therefore, the observed product ion spectrum is assumed to be a linear combination of the corresponding fragmentation spectra in the database. Note that, in practice, we must also accommodate the possibility that ions of multiple charge state and isotope composition may also fall in the isolation window. This is a technical detail that does not change the general form of the algorithm.

Suppose that there are $K$ candidate product ion spectra in the database corresponding to a given precursor isolation window. We construct a database matrix **D** containing $N$ rows and $K$ columns as shown in Eq. 1,

$$\mathbf{D} = \begin{bmatrix} \mathbf{d}_1 & \mathbf{d}_2 \cdots \mathbf{d}_K \end{bmatrix} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \begin{bmatrix} d_{n,k} \end{bmatrix}_{N \times K} \qquad (1)$$

in which each column, $\mathbf{d}_k$ (for $k$=1...$K$) is a database (spectral library) spectrum represented as a (column) vector having $N$ entries, $d_{n,k}$ (for $n$=1...$N$). The entry $d_{n,k}$ is the observed intensity of the $k^{th}$ product ion spectrum at the $n^{th}$ $m/z$ "position", this intensity possibly being an integrated intensity over a narrow mass range. It is necessary that the product ion spectra be normalized so that the entries at corresponding positions in the vector represent equivalent mass positions.

Suppose that s denotes a product ion spectrum that we wish to interpret in terms of the spectral library **D**. In particular, we wish to find the weighted sum of database spectra that most closely approximates s. An arbitrary linear combination, **s'**, of the database spectra is given by Eq. 2, viz.

$$\mathbf{s'} = \sum_{k=1}^{K} x_k' \mathbf{d}_k = \begin{bmatrix} \mathbf{d}_1 & \mathbf{d}_2 \cdots \mathbf{d}_K \end{bmatrix} \begin{bmatrix} x_1' \\ x_2' \\ \vdots \\ x_K' \end{bmatrix} = \mathbf{D}\mathbf{x'} \qquad (2)$$

in which the column vector **x'** is an arbitrary vector of $K$ abundances, not necessarily optimal. The goal is to determine an optimal estimate of the abundances, which we denote by $\hat{\mathbf{x}}$, where we interpret the product ion spectrum s as a mixture of database spectra. According to our model, $\hat{\mathbf{x}}$ is also an estimate of the abundances of the precursors, represented by these database spectra, that occur in the unknown mixture that gave rise to the observed product ion spectrum s. The optimality criterion used here is the sum of weighted squared differences between the components of **s** and **s'**, denoted by the scalar quantity $e$ in Eq. 3, below. The sum is over the data samples in the observed spectrum **s**. The quantity $e$ is the squared length of the vector difference between **s** and **s'**. The squared length of a vector can be written as the transpose of a vector times itself.

$$e = \sum_{n=1}^{N}(s'_n - s_n)^2 = (\mathbf{s}'-\mathbf{s})^{\mathrm{T}}(\mathbf{s}'-\mathbf{s}) = (\mathbf{D}\mathbf{x}'-\mathbf{s})^{\mathrm{T}}(\mathbf{D}\mathbf{x}'-\mathbf{s}) \qquad (3)$$

Minimizing the squared length of the vector is equivalent to minimizing the length of the vector, and more convenient mathematically. We assume correspondence between the mass positions represented by the entries of s and the database product ion spectra so that a vector difference between the quantities is meaningful.

Determining the set of parameter values that minimizes the sum of squared differences between a model and observed data is equivalent to maximum-likelihood estimation in the special case where the observed data is assumed to be the outcome of a random process in which an "ideal" model is corrupted by additive, white Gaussian noise. This is a convenient model, but not perfectly applicable to the current problem. Most of the random variation seen in product ion spectra is due to ion counting statistics. Fortunately, the variations of measurements at different mass positions in the spectra are independent. However, the variations are not identically distributed. In counting statistics, the variance is equal to the count intensity. When the ion counts are relatively large (i.e. greater than 50 ions), the distribution of observed values can be accurately approximated by a Gaussian distribution where the variance is set equal to the count intensity. Because the underlying count intensity is unknown, we can approximate the variance by the observed intensity, rather than the count intensity, without introducing significant distortion in our error metric. To take into account, the differences in the variance across samples, we modify our error metric as in Eq. 4 as

$$e = \sum_{n=1}^{N}\left(\frac{s'_n - s_n}{\sigma_n}\right)^2 = (\mathbf{s}'-\mathbf{s})^{\mathrm{T}}\mathbf{W}(\mathbf{s}'-\mathbf{s}) = (\mathbf{D}\mathbf{x}'-\mathbf{s})^{\mathrm{T}}\mathbf{W}(\mathbf{D}\mathbf{x}'-\mathbf{s}) \qquad (4)$$

where $\mathbf{W}$ is an $N$-by-$N$ ($N \times N$) diagonal matrix of weighting factors wherein each diagonal entry $W_{nn}$ is defined as $W_{nn} = 1/\sigma_n^2 = 1/s_n$ and all off-diagonal entries are zero. Additional sources of variation can be taken into account by modifying $\sigma_n$ appropriately, but in this application, $\sigma_n$ is dominated by counting statistics. In any case, the variation is encapsulated in matrix $\mathbf{W}$, which is constant with respect to the estimated abundances.

Next, we derive the optimal vector of estimated abundances, denoted by $\hat{\mathbf{x}}$. To find the optimal value, we evaluate the derivative of $e$ with respect to each component of $\mathbf{x}'$. Because $\hat{\mathbf{x}}$ minimizes $e$, the derivative of $e$ evaluated at $\hat{\mathbf{x}}$ must be zero. Therefore, we set the derivative of e evaluated at $\hat{\mathbf{x}}$ to zero (specifically, the null vector $\mathbf{0}$) as indicated in Eq. 5, below.

$$\begin{bmatrix} \partial e / \partial x_1 \\ \partial e / \partial x_2 \\ \vdots \\ \partial e / \partial x_K \end{bmatrix}\Bigg|_{\hat{x}} = \mathbf{D}^{\mathrm{T}}\mathbf{W}(\mathbf{D}\hat{\mathbf{x}} - \mathbf{s}) = \mathbf{0} \qquad (5)$$

Rearranging Eq. 5 produces Eq. 6, the desired linear matrix equation for the optimal estimate of the abundance vector.

$$\left(\mathbf{D}^{\mathrm{T}}\mathbf{W}\mathbf{D}\right)\hat{\mathbf{x}} = \mathbf{D}^{\mathrm{T}}\mathbf{W}\,\mathbf{s} \qquad (6)$$

The above derivation is based on decomposing a single observed or unknown multiplex spectrum, represented by vector s into its various components as are recorded in a spectral library. It may be advantageous, in some computing architectures to perform the decompositions of multiple spectra at once. Let matrix $\mathbf{S}$ denote a collection of $Q$ product-ion spectra, all acquired using the same precursor isolation window, formed by stacking the individual product ion spectra as column vectors, i.e. $\mathbf{S} = (\mathbf{s}_1, \mathbf{s}_2, \dots \mathbf{s}_Q)$. Then we replace vectors s and $\hat{\mathbf{x}}$ in Eq. 6 with matrices $\mathbf{S}$ and $\hat{\mathbf{X}}$ respectively in Eq. 7, so as to yield

$$\left(\mathbf{D}^{\mathrm{T}}\mathbf{W}\mathbf{D}\right)\hat{\mathbf{X}} = \mathbf{D}^{\mathrm{T}}\mathbf{W}\mathbf{S} \qquad (7)$$

in which $\hat{\mathbf{X}}$ is a matrix of $Q$ column vectors, each with $K$ entries. The entry $\hat{\mathbf{X}}_{kq}$ contains the optimal estimate for the abundance in observed product ion spectrum $\mathbf{s}_q$ of the precursor represented by in the database by product ion spectrum $\mathbf{d}_k$.

To simplify analysis, consider the special case where $\mathbf{W} = \mathbf{I}$, where $\mathbf{I}$ is the $N \times N$ identity matrix. In this case, we do not consider the differences in variance between observed values in the product ion spectrum $\mathbf{s}$. Then Eq. 6 reduces to

$$\left(\mathbf{D}^{\mathrm{T}}\mathbf{D}\right)\hat{\mathbf{x}} = \mathbf{D}^{\mathrm{T}}\mathbf{s} \qquad (8)$$

Let $\mathbf{A}$ denote the $K \times K$ matrix $\mathbf{D}^{\mathrm{T}}\mathbf{D}$ and let $\mathbf{b}$ the K-vector $\mathbf{D}^{\mathrm{T}}\mathbf{s}$. The entry $A_{k'k}$ is equal to $\mathbf{d}_{k'}^{\mathrm{T}}\mathbf{d}_{k}$, or equivalently the dot product between database product ion spectra $\mathbf{d}_k$ and $\mathbf{d}_{k'}$. The entry $b_k$ is equal to $\mathbf{d}_{k}^{\mathrm{T}}\mathbf{s}$, or equivalently the dot product between database product ion spectrum $\mathbf{d}_k$ and the observed product ion spectrum s. If $\mathbf{d}_k$ and $\mathbf{d}_{k'}$ are appropriately normalized, $A_{kk'}$ is the correlation coefficient between vectors $\mathbf{d}_k$ and $\mathbf{d}_{k'}$. A trivial example is when $\mathbf{A}$ is the identity matrix. In this case, there is no overlap between database entries - that is, the product ion spectra have no overlapping peaks. In this case, $\hat{\mathbf{x}}_k = b_k$ or, in other words, the optimal estimate for the abundance of the $k^{th}$ database spectrum, $\mathbf{d}_k$, in the observed product ion spectrum is the dot product between $\mathbf{d}_k$ and s. In general, the estimated abundance of the $k^{th}$ database spectrum in the observed product ion spectrum depends not only upon its dot product with the observed spectrum, but also its dot product with the other spectra in the database. In the extreme case, suppose $\mathbf{A}$ has an entry whose value approaches one. In that case, product ion spectra $d_k$ and $\mathbf{d}_{k'}$ are nearly indistinguishable, and so the estimated values are highly sensitive to small amounts of noise. Therefore, to ensure stable estimates, it is important to construct a database that contains distinct product ion spectra, avoiding duplicate or very similar entries.

*Analysis*

Error analysis of the abundance estimates is now discussed. If the observed ion spectrum is exactly a mixture of one or more product ion spectra, then the estimated abundances will be exactly the coefficients of the mixture. For example, consider $\mathbf{s} = \mathbf{D}\mathbf{x}$ where $\mathbf{x}$ is a vector of the true abundances in the mixture. Then the vector of estimated abundances $\hat{\mathbf{x}}$ is equal to the vector of true abundances, wherein

$$\left(\mathbf{D}^{\mathrm{T}}\mathbf{D}\right)\hat{\mathbf{x}} = \mathbf{D}^{\mathrm{T}}\mathbf{s} = \mathbf{D}^{\mathrm{T}}\left(\mathbf{D}\mathbf{x}\right) = \left(\mathbf{D}^{\mathrm{T}}\mathbf{D}\right)\mathbf{x} \qquad (9)$$

If we assume that the product ion spectrum is the outcome of a random process in which a mixture of product ion spectra from the database is corrupted by additive white, Gaussian noise, then we have Eq. 10, as follows.

$$\left(\mathbf{D}^{\mathrm{T}}\mathbf{D}\right)\hat{\mathbf{x}} = \mathbf{D}^{\mathrm{T}}\mathbf{s} = \mathbf{D}^{\mathrm{T}}\left(\mathbf{D}\mathbf{x} + \mathbf{n}\right) = \left(\mathbf{D}^{\mathrm{T}}\mathbf{D}\right)\mathbf{x} + \mathbf{D}^{\mathrm{T}}\mathbf{n} \qquad (10)$$

The vector of estimated abundances can be written as the vector of true abundances plus an error term, as given by Eq. 11.

$$\hat{\mathbf{x}} = \mathbf{x} + \left(\mathbf{D}^{\mathrm{T}}\mathbf{D}\right)^{-1} \mathbf{D}^{\mathrm{T}}\mathbf{n} = \mathbf{x} + \Delta \qquad (11)$$

The error term is a linear transformation of a zero-mean Gaussian random variable, and therefore, is itself a zero-mean Gaussian random variable. Because the error is zero-mean, we say that the estimator is unbiased. A zero-mean Gaussian random variable is characterized by its covariance matrix. The covariance is given in Eq. 12 below as

$$K_\Delta = \left\langle \Delta \Delta^{\mathrm{T}} \right\rangle = \left(\mathbf{D}^{\mathrm{T}}\mathbf{D}\right)^{-1} \mathbf{D}^{\mathrm{T}} \left\langle \mathbf{n}\mathbf{n}^{\mathrm{T}} \right\rangle \mathbf{D} \left(\mathbf{D}^{\mathrm{T}}\mathbf{D}\right)^{-1} = \sigma^2 \left(\mathbf{D}^{\mathrm{T}}\mathbf{D}\right)^{-1} \qquad (12)$$

where $\sigma^2$ is the sample variance of the noise.

The matrix $(\mathbf{D}^{\mathrm{T}}\mathbf{D})^{-1}$ can be considered as a gain factor which expresses how much the input noise gets amplified in producing the abundance estimates. In the simple case, where $\mathbf{D}^{\mathrm{T}}\mathbf{D}$ is identity (no overlap between database spectra), the amplification is one, meaning that the abundance estimates have the same variance as the individual samples in the spectrum, and are mutually independent. In general, the inverse eigenvalues of $\mathbf{D}^{\mathrm{T}}\mathbf{D}$, or equivalently the eigenvalues of $(\mathbf{D}^{\mathrm{T}}\mathbf{D})^{-1}$, which can be computed in advance, determine the amplification of noise. When the input noise is expressed in terms of components in the directions of the eigenvectors of $\mathbf{D}^{\mathrm{T}}\mathbf{D}$, each noise component is amplified by the corresponding inverse eigenvalue. Certain noise components, e.g. in the direction of similar spectra, undergo large amplification of noise causing unstable estimates in the abundance of such spectra. While other directions, i.e. in the direction of highly distinct spectra, may undergo essentially no amplification, leading to relatively stable estimates of these abundances. In any case, the estimation errors can be estimated in advance from the spectral database. This admits the possibility of designing optimal experimental protocols that produce tolerable levels of errors or optimal interpretation of existing experimental protocols.

If the matrix $\mathbf{D}^{\mathrm{T}}\mathbf{D}$ is nearly singular, then the abundance estimates will have large errors. The matrix $\mathbf{D}^{\mathrm{T}}\mathbf{D}$ will be singular when one of the rows of the matrix can be written as a sum of the other rows. A trivial case is a matrix that contains two identical rows. The matrix is said to be ill-conditioned when it is nearly singular. This will happen when the database contains two spectra from the same precursor or contains a multiplex of spectra from precursors represented in the database. Some care must be taken to avoid this condition. If the database matrix $\mathbf{D}$ has more entries than sample values per entry, i.e. $K > N$, the matrix $\mathbf{D}^{\mathrm{T}}\mathbf{D}$ will be singular.

Note that the resolution of spectra in the library has a significant effect on the overlap scores in the matrix $\mathbf{D}^{\mathrm{T}}\mathbf{D}$. For example, at low resolution, peak overlaps between similar masses increase, resulting in larger overlap scores between distinct database entries, i.e. "off the diagonal" of the matrix $\mathbf{D}^{\mathrm{T}}\mathbf{D}$. Pairs of spectra that have large overlaps, for example, either because of inherent similarity or insufficient resolution in the acquired spectra, are difficult to discriminate, and also reducing the overall discriminating power of the estimator by amplifying input noise.

The error covariance matrix can be used to determine the appropriate threshold for accepting low abundance ions versus setting noisy fluctuations in estimated abundances to zero. For example, if noise (i.e. a spectrum that does not overlap with any database entries) is presented to the estimator, the desired abundance vector would be zero, but in fact, the computed abundance vector is a random Gaussian vector. The mean value of the vector is zero but component values will fluctuate about zero due to the input noise, which is amplified and propagated by the estimator. The distribution of scores can be calculated for each database entry. The probability that any given score exceeds some arbitrary value (i.e. a threshold) can also be computed.

Therefore, a threshold can be chosen below which component scores can be discarded as noise. The threshold can be chosen so that only a small fraction of false positives are accepted. This false positive rate can be specified and used to compute the relevant threshold for detection.

At a given false positive rate, the detection sensitivity can also be calculated. Sensitivity depends upon signal-to-noise, as expected, but also depends upon the extent to which various components in the database can be discriminated from one another. This rather qualitative description of how sensitivity depends upon the database is exactly specified quantitatively by the error covariance matrix $(\mathbf{D}^{\mathrm{T}}\mathbf{D})^{-1}$.

*Applications*

The techniques described in this disclosure can be applied to either intentional or unintentional multiplexing. Intentional multiplexing refers, for example, to the sequential accumulation of multiple precursors in an ion trap, simultaneous fragmentation of all precursors, and simultaneous analysis. Intentional multiplexing can be used, for example, to exploit the large spectral bandwidth of Orbitrap® mass analyzers (commercially available from Thermo Fisher Scientific of Waltham Massachusetts USA) relative to the relatively low complexity of a single $MS^2$ spectrum.

Unintentional multiplexing refers to the isolation of multiple precursors which happen to lie in the same isolation window as a single targeted precursor. This situation is unavoidable in complex mixtures, given that isolation windows are typically one Dalton or wider. The techniques taught in this document can be used for identification of additional precursors, even when it is believed that a single precursor has been isolated. In that case, only one precursor should have an estimated abundance significantly different from zero.

In some implementations, it may be advantageous to perform $MS^2$ identifications in real-time on an embedded system. Graphic processing units (GPUs) are ideally suited for carrying out the required linear algebraic computations quickly and at relatively low cost.

*Constructing the Spectral Library and Candidate Lists for Precursor Identification*

There are two general approaches to identifying precursors from product ion spectra. The first involves matching the observed spectrum to entries in a spectral library database. The alternative is product ion spectrum prediction. In theory, observed product ion spectra provide substantial information about the identity of a precursor compound. Product ion spectra typically contain many detectable peaks. The collection of mass positions and intensities of these peaks provide a distinctive fingerprint of the precursor compound. Furthermore, the observed product ion spectrum is highly reproducible. Taking these two properties together, it is unlikely that random variations that affect the acquisition of a product ion spectrum would cause one precursor compound to be mistaken for a different precursor compound.

Despite the potential utility of spectral libraries for identification, conventional spectral libraries do not guarantee accurate, confident precursor identification. Several problems with spectral libraries are addressed in this patent application. Most importantly, spectral libraries are substantially incomplete. While it is trivial to acquire a product ion spectrum on a modern mass spectrometer, it is relatively laborious to prepare purified precursors to submit to the mass spectrometer to generate the pure product ion spectra that are needed for use a spectral library. General-purpose spectral libraries may contain thousands of compounds, but typically do not provide extensive coverage of the molecules encountered in many, highly-specific applications. If an analyzed precursor does not appear in the spectral library, the search will result, at best, no result, and at worst, an incorrect identification.

Even in the case where an analyzed precursor appears in the library, the library entry for that precursor may be a poor match to the observed spectrum because the two spectra have been acquired on different instruments and/or using different fragmentation conditions. For example, resonant collisionally induced dissociation in an ion trap, commonly called CID or less commonly RECID, may produce a significantly different product ion spectrum than collisionally induced dissociation where ions are accelerated to high-energy axial energy before entering a quadrupole (HCD). In RECID, the precursor resonantly absorbs energy based upon its mass-to-charge ratio and generates primary fragments upon colliding with neutral gas molecules. The primary fragments are not in resonance by virtue of their change in mass-to-charge, quickly lose energy, and do not produce secondary fragments. Conversely, in HCD, primary fragments may retain high kinetic energy and give rise to secondary fragments.

Diverse methods of fragmentation such as electron transfer dissociation (ETD), ultraviolet photodissociation (UVPD), and infrared multiple photon dissociation (IRMPD) rely upon completely different fragmentation mechanisms. Each of these fragmentation methods produces product ion spectra with distinctive properties that depend upon differing aspects of the precursor structure and reactivity.

Even when restricted to the same general type of fragmentation, differences in the experimental parameters can cause significant variations in the distribution of product ions. For example, in CID, increasing the pressure in the collision cell tends to favor multiple, sequential fragmentation events. A similar effect can be seen by lengthening the reaction time in ETD. Increasing the collision energy can favor different fragmentation pathways in CID than at lower energy.

The numerous difficulties with spectral libraries have led many practitioners to favor the alternative approach in which a product ion spectrum or, more commonly, a list of product ion masses is predicted from a precursor molecule of known structure. The primary advantage of this method is that an algorithm can generate a predicted spectrum for essentially any molecule that can be conceived, eliminating the need to synthesize and purify the molecule for analysis. The method is used to its greatest advantage in bottom-up proteomics. In such an application, the product ions of tryptic peptides formed by CID spectra are primarily b- and y-type ions that can be easily and reliably enumerated.

The disadvantages of product ion spectrum prediction, however, are significant: prediction quality is poor. It is difficult to predict the most abundant product ions for most classes of molecules. Even in cases where product ions can be predicted, intensity information in the observed product ion spectrum usually cannot be exploited. Large uncertainty in the prediction and the failure to use product ion intensities to discriminate precursors often result in mistaking one precursor compound for another.

Several deficiencies in spectral libraries that limit their utility in identification can be overcome simultaneously by enabling a mass spectrometer to use each product ion spectrum it acquires to generate its own spectral library. For confident identification, it is critical to collect analytic product ion spectra under essentially the same conditions for which the corresponding spectral library entry was collected. The best way to ensure this correspondence is to construct the spectral library on the same instrument where the subsequent analysis will be performed. In addition, it is necessary to standardize the experimental parameters to eliminate unnecessary variation. For example, the collision energy can be set as a deterministic function of the isolation window. If an instrument is enabled for multiple types of fragmentation, then separate libraries should be maintained for each fragmentation type.

A comprehensive approach in which every spectrum acquired on the mass spectrometer is used for automatic spectral library construction makes it possible to generate libraries that are essentially complete for a given application in a relative short amount of time and without any burden on the user. For example, if a mass spectrometer is acquiring product ion spectra at a rate of 10Hz, the number of spectra acquired in an hour is $10 \times 60 \times 60 = 36,000$; the number of spectra that may be acquired in a day is $36,000 \times 24 = 864,000$. It is thus possible to collect a product ion spectrum on nearly every detectable molecule in a class of samples, e.g. a collection of human proteomes in a clinical trial, in a matter of days or weeks.

Although the number of product ion spectra obtained over the lifetime of a mass spectrometer may number in the billions-i.e. a million a day for thousands of days-the size of the spectral library depends only upon the number of unique precursors it detects. The number of unique detectable molecules detected by a mass spectrometer is typically several orders of magnitude smaller. If a database contains one million product ion spectra and each spectrum requires a kilobyte of storage (i.e. four bytes for mass and four bytes of intensity for a few dozen peaks plus annotation), the memory required to store the database is one gigabyte. Thus, typical databases that encapsulate a complete record of every precursor a mass spectrometer will ever encounter can be stored locally and accessed rapidly.

A distinctive aspect of the automatically generated spectral library is that it contains an entry for every precursor that is detected, even if an identification has not yet been made. A novel precursor is typically added to the library without an annotation that identifies it. When the precursor is subsequently presented to the mass spectrometer, it is matched to the corresponding library entry, but not, strictly speaking, identified, unless that entry has been annotated.

Most of the precursors in the database may never need to be identified. For example, it is sufficient to be able to match a compound to the unidentified entry in the database to allow comparative analysis of multiple samples. However, if a particular precursor compound appears to have some significance, e.g. as a potential biomarker whose abundance stratifies patient response to some therapeutic intervention, then some additional effort can be taken to annotate the entry. The current inventive method does not address how that annotation is performed. However, once an entry is annotated, the corresponding precursor is identified each time it is encountered, simply by matching it to the annotated entry.

A key enabling aspect of the inventive method is the ability to compile a library in which each precursor it encounters is represented by a single entry representing the product ion spectra that would be acquired if the precursor were purified and subsequently fragmented. The analytic method for interpretation of the product ion spectra derived from mixtures of precursors described above is essential to the automatic construction of a suitable spectral library.

Consider the case where a product ion spectrum is acquired from a mixture of precursors that have been previously seen by the mass spectrometer and for which accurate spectral library entries exist. The acquired spectrum is projected onto the spectral library, and the precursors are correctly identified and quantified. The estimated mixture of identified precursors can be used to form a model product ion spectrum and compared against the observed spectrum. The residual difference between the spectra can be analyzed for the presence of additional novel precursor components. In this case, the residue would be judged to be typical noisy variations and discarded.

Now, consider the case where a product ion spectrum is acquired from a mixture of precursors which includes a compound that is not represented in the spectral library. In this case, the residual difference between a model product ion spectrum constructed from the extracted components and the observed spectrum would be significant. One might then hypothesize that the residue contains one or more novel precursors.

The threshold below which residual components should be discarded depends upon the reproducibility of the product ion spectra. Consider a case where a given known primary precursor is mixed with a small amount (e.g. 5%) of an unknown secondary precursor. Suppose the product ion spectrum of the known primary precursor has a typical variation of 1%. Then, the difference between the product ion spectrum and the library entry for the known primary precursor is significant; the residue is unlikely to be explained by variations in the appearance of the product ion spectrum of the primary precursor. Conversely, if the secondary precursor is present at 1% abundance, its presence may not be detected and the residue may be considered as typical variation in the product ion spectrum of the primary precursor.

An LC-MS experiment provides a convenient way to verify putative novel precursors and to purify multiple novel precursors from the residue after known precursors are extracted. Correlation between the time profiles of pairs of product ions or between a product ion and a putative precursor can be used to match product ions to a precursor and have been described in the art.

For example, a product ion spectrum acquired during an LC-MS experiment reflects a mixture of precursors that happen to elute at the same time. If we could obtain the elution profile of each precursor, we would see all precursors were eluting at the time when the product ion spectrum was acquired. Although the profiles overlap at this time, the profiles are, in general, not identical. For example, they may be shifted slightly in time. In addition, each product ion derived from a given precursor has a profile that is essentially identical, except for statistical fluctuations, to the profile of its precursor. Therefore, within a collection of product ion spectra representing a mixture of two or more precursors obtained sequentially over a short duration of time in a LC-MS run, we expect to see one subset of product ions whose abundances move up and down in concert with each other and the precursor elution profile, while another subset of product ions move up and down together in a different pattern, slightly shifted in time.

To ensure the quality of the spectral library entries, we can enforce the rule that we do not add a putative precursor to the spectral library, unless the elution profiles of its product ions can be matched to or correlated with the precursor profile that can be directly observed in the precursor spectrum. We expect to be able to detect trace compounds in a product ion spectrum even when are not directly observed in the precursor spectrum. However, one can set a higher standard for including these in the library when they are observed for the first time.

We have mentioned that the precursor may or may not be observed in the precursor spectrum that is usually obtained immediately before triggering a product ion spectrum. When one selects an isolation window, there necessarily exists coarse, but definitive information about the precursor mass: that is, the precursor mass is inside the isolation window. Even with this coarse information about the precursor mass, one need not project the product ion spectrum onto the entire spectral library. Instead, one can form a candidate list from the spectral library whose precursor masses lie in the given window. These candidate lists are most conveniently generated by keeping the spectral library entries sorted by precursor mass. In the case of sequential multiplexing, the database would be constructed by concatenating lists of precursors contained in each of multiple isolation windows.

When the set of possible isolation windows can be enumerated in advance, e.g. 1-Da wide windows at each nominal mass between 1-2000, the spectral library can be partitioned in advance. For example, a database of one million entries might be divided into 2000 mini-databases (one for each nominal mass up to mass 2000) each containing, on average, 500 entries. For each of these sub-libraries, the matrix $\mathbf{D}^T\mathbf{D}$ can be stored and pre-factored (i.e. by LU decomposition). If there are K entries in the database, the computational complexity of solving the matrix equation for the abundance estimates is reduced from $O(K^3)$ to $O(K^2)$ when an LU decomposition has been pre-computed. Together, using the isolation window to reduce the list of candidate precursors and pre-factorization of the matrices make it possible to interpret mixed spectra of arbitrary complexity in real time.

The act of assuming that the only information about the precursor mass is given by the isolation window is a "blind" approach, because it does not consider information in the precursor spectrum. An alternative approach to the "blind" decomposition is constructing a small list of candidate precursors in real time based upon accurate mass measurements of detected peaks that lie in the isolation window in the precursor spectrum. On an accurate mass instrument, the precursor mass can be confined to a mass range that can be three orders of magnitude smaller than isolation window (i.e. mDa vs. Da), thus reducing the list of precursor candidates by a similar factor. Guided by this information, a database is constructed by concatenating lists of precursors whose masses lie within a confidence interval of one of the estimated masses of detected precursors.

Not only is the calculation much faster when a small subset of the spectral library is used, but the false positive rate is also reduced. The disadvantage of this method is that it precludes detection of low abundance precursors that were not detected in the precursor scan. However, this disadvantage is relatively small in cases where an isolated precursor has such high abundance that detection of additional precursors in the product ion spectrum would require excessively high dynamic range.

Regardless of how a list of precursor candidates is generated from the spectral library, the error covariance matrix can be computed (or pre-computed) for any specific list of candidates. The error covariance matrix indicates how much noise or variation in the product ion spectrum will be amplified in generating the abundance estimates. At a certain level of error, one cannot distinguish whether a given precursor is present at low abundance or completely absent, leading to false-positive and false-negative identification errors. A real-time decision can be made regarding how much acquisition time would be necessary to make a correct identification based upon the list of candidates. In some cases, an easier target might be preferred if the acquisition time required for identification at a particular confidence level is judged to be lower.

*Example*

A test library was constructed by performing the following operations: (a) reading a list of spectra contained in one or more files; (b) sorting the spectra by precursor mass-to-charge (*m/z*) ratio; (c) building library segments according to a user-defined precursor window step size; (d) assigning ions of the MS$^2$ spectra to various bins according to a user-defined step size (resolution); (e) building qualified library segments by computing the $\mathbf{D}^T\mathbf{D}$ matrix for all spectra in a given precursor range, examining this matrix for pairs of highly correlated spectra (redundant entries), consolidating redundant entries, and re-computing the matrix; (f) computing and storing the LU factorization and the inverse of the $D^TD$ matrix. Neither the sample-weighting scheme to account for spectral variations due to ion counting statistics nor the detection threshold for ignoring calculated abundances that are not statistically different from zero were implemented in this example.

After the library was constructed, additional spectra were searched against this library by solving for $\hat{x}$ in Eq. 8. The graphics processing unit (GPU) was utilized for all matrix operations. We performed the $MS^2$ de-multiplexing calculations using two test cases: Case #1 a set of 1.1 million $MS^2$ spectra from an LC-MS analysis of yeast proteomic samples on a Q-Exactive™ mass analyzer instrument (commercially available from Thermo Fisher Scientific of Waltham Massachusetts USA and including a quadrupole mass filter for precursor selection, a Higher Collision Energy Dissociation fragmentation cell and a high-resolution accurate-mass Orbitrap® mass analyzer for analysis and detection) and; Case #2 synthetically multiplexed spectra formed by summing observed $MS^2$ spectra and adding random noise.

In the first case, we aggregated a large set of $MS^2$ spectra of yeast samples produced on a Q-Exactive™ instrument in which fragments ions were produced in an HCD cell. Over 1.1 million such $MS^2$ spectra were collected and searched via the Mascot software search engine (a conventional search engine that is able to identify proteins from mass spectrometry data) against a yeast protein database. Because a functional spectral library may be presumed to be curated for quality and relevance, we used the Mascot search as a filter to approximate such curation. Spectra that yielded peptide identifications at a false positive rate of 5% or better were retained and written to text files, yielding about 150,000 such spectra. Of the above spectra, 111,604 spectra were read from files to build a library, while 44,263 spectra were kept in reserve to serve as queries against the library. Spectra were binned at 0.1 *m/z* resolution and normalized to unit vectors. Each vector spans a mass range from 0 to 2000 Da, and thus contains 20,000 sample values.

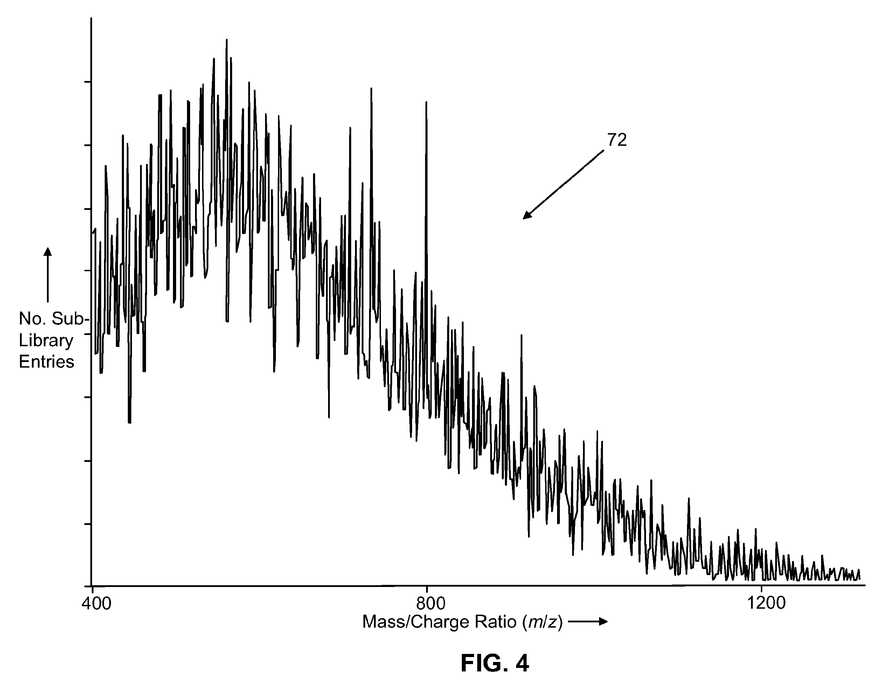

The library was partitioned into sub-libraries representing 1 *m/z* unit (Da/e) precursor isolation steps. This relatively coarse partitioning of the library enables "blind" de-multiplexing. In blind de-multiplexing, it is possible, in theory, to detect precursors in an $MS^2$ spectrum, even when the precursor is not detected in $MS^1$ scan. Alternatively, one can use the accurate mass measurement of the detected precursor(s) in the isolation window to limit the search to a very small number of candidate precursors. The difficulty of the de-multiplexing problem grows non-linearly with the number of candidates in the library partition. We chose blind de-multiplexing as a test case to demonstrate the power of the method. The distribution of library sizes is shown as graph **72** in FIG. 4.

The library was formed from non-redundant spectra, with the intent of retaining one copy of a spectrum from each distinct precursor. Redundant spectra were consolidated as follows: the matrix $D^TD$ containing all pairwise dot products of spectra (correlation coefficients) was examined for entries above a threshold. Highly correlated pairs were aggregated, reduced to distinct sets, and averaged. Single averaged spectra then replaced their "parent" spectra. Consolidation of a very large number of redundant entries explains why the final count of spectra (27,714) is much smaller than the size of the starting set.

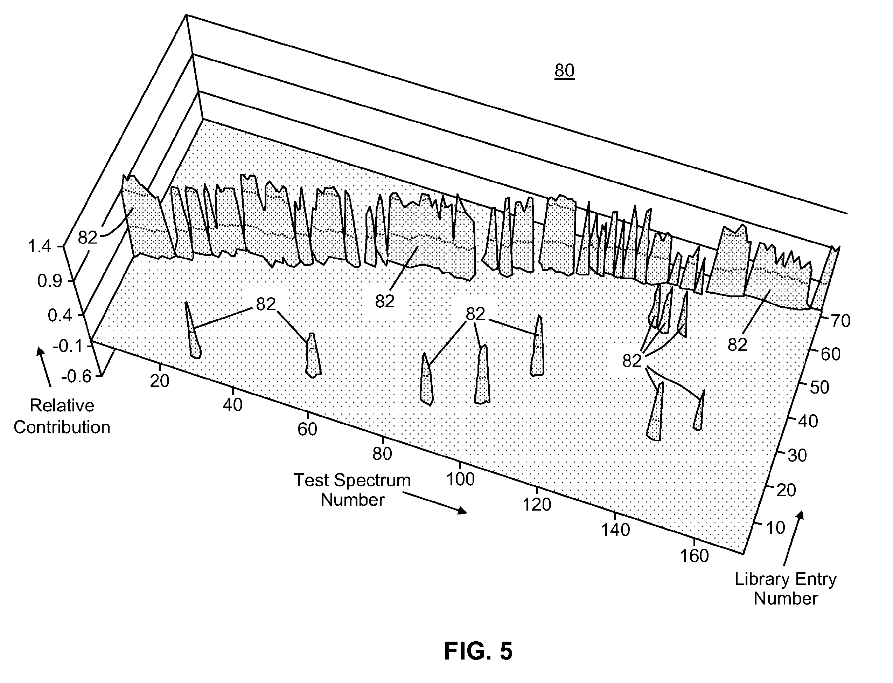

The 44,263 test spectra were sorted by precursor *m/z* and searched against the library, taking about two minutes on a laptop computer. An example results matrix is shown in FIG. 5 as a 3D contour plot **80** comprising 3D surface **82,** representing the search results of all test spectra of the precursor ions having *m/z* values in the range 436-437 Da. This results set was chosen arbitrarily. This segment of the results consists of a set of 172 vectors, corresponding to 172 test spectra. Each vector is 72 values long, where each value is the estimated "abundance" of a respective library spectrum component. In most cases, each results vector contains a single large abundance value, indicating very high correlation with a single library spectrum, in other words, an identification.

The above calculations attempt to express the observed spectrum as a mixture of precursors. Because the spectra were pre-filtered by retaining only those spectra that produced high-confidence Mascot identifications, we expect most of the spectra to contain one (pure) component. The calculated results indicate that, in most cases, the methods indicated (presumably correctly) that one pure precursor was present. These values can be seen to form a near diagonal wall (the predominant feature of the surface **82)** across the contour plot. The continuity between hits reflects the fact that both sets were sorted by precursor *m/z* prior to searching. Significant hits off of the diagonal can represent incorrect precursor assignment in the test data, or the presence of multiple peptides in the test spectra (multiplexed spectra).

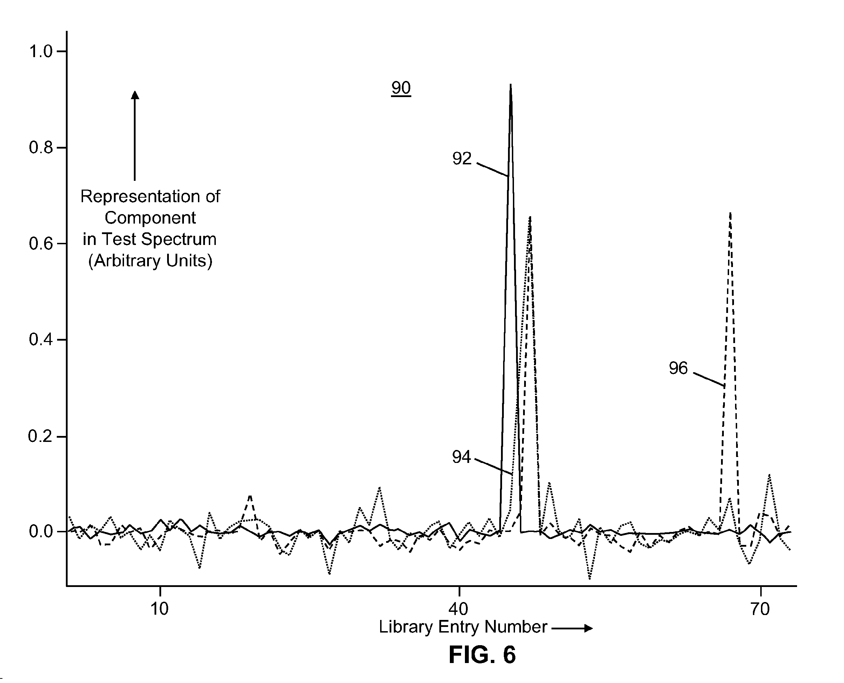

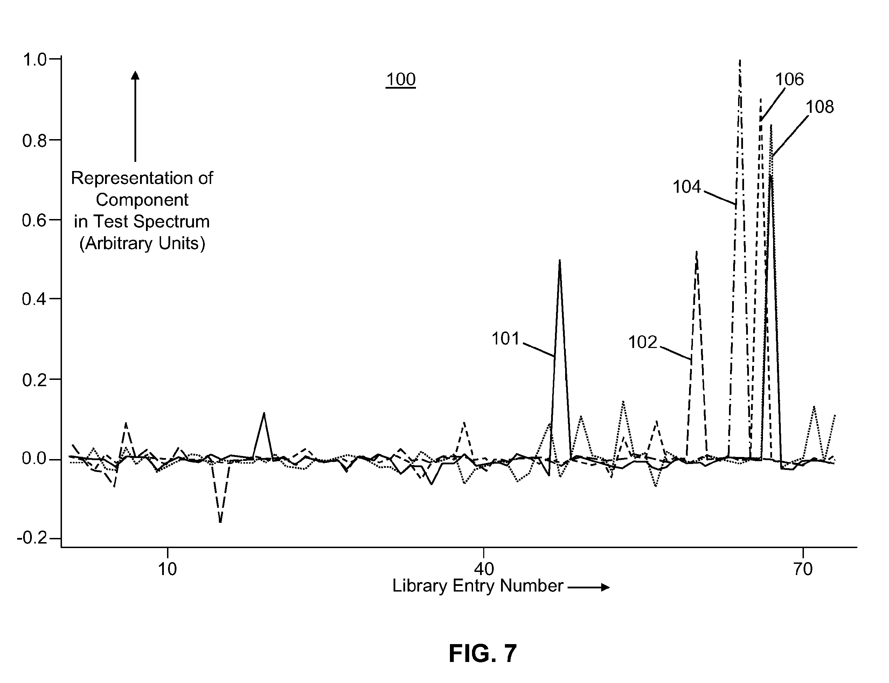

A visual inspection of the results illustrated in FIG. 5 suggests that 90% or greater of test spectra contained at least one significant match against the database. Manual inspection also revealed numerous cases of putative multiplexed detection, examples of which are shown in FIGS. 6 and 7. Both FIGS. 6 and 7 represent different subsets of the data of FIG. 5 pertaining to different respective test spectra. The line plots in these figures represent the relative contributions of different library components within each test spectrum, as calculated by the decomposition routine. Graph **90** in FIG. 6 shows depictions of three results vectors (each results vector corresponding to a different respective spectrum) from the search against the library segment 709-710 (*m/z*). Results vector plot **92** (solid line) gives a clear hit against a single library result, as does the results vector plot **94** (dotted line). In contrast, the vector plot **96** gives strong hits against the library component present in the results vector **94** and against an additional component, indicating that it very likely corresponds to a multiplex spectrum. Graph **100** in FIG. 7 contains a similar set of results vectors calculated for five different respective test spectra. The spectrum associated with results vector **101** (solid line) can be seen to contain two major components. By contrast, the spectra associated with results vectors **102** (long-dashed line), **104** (dashed-dotted line), **106** (small-dashed line) and **108** (dotted line) all give a clear hit against a single respective library component.

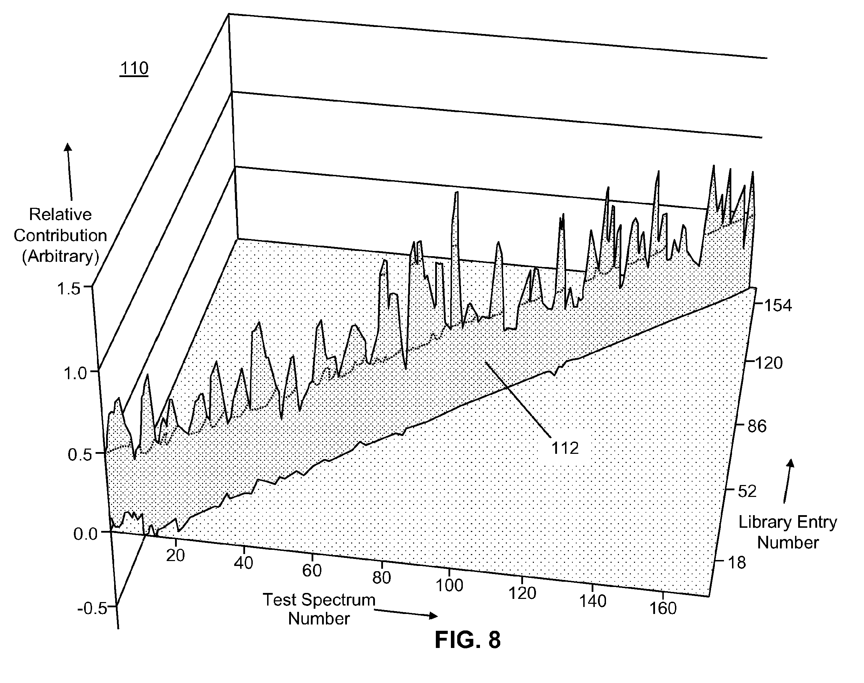

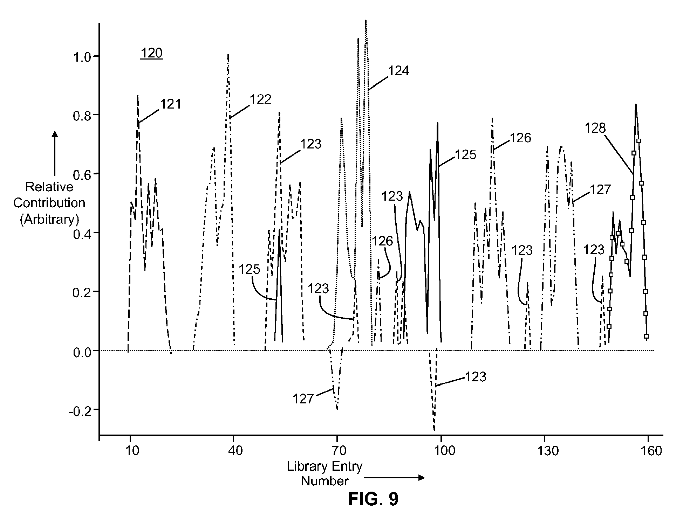

Detection of unintentionally multiplexed spectra is one major potential application of this approach; another is the decomposition of intentionally multiplexed spectra, that is spectra derived from multiple sequential isolations of different precursor species, followed by combined analysis of their fragments. To attempt to explore the suitability of our approach for such data, we took a set of MS$^2$ spectra in which fragmentation was accomplished by HCD, such as in the system **15** illustrated in FIG. 1, and intentionally composited sets of sequential spectra. In the results shown in FIGS. 8-9, every set of ten sequential spectra in a file were composited, such that the resulting spectrum #1 contains a mixture of spectra #1-#10, spectrum #2 contains a mixture of spectra #2-#11, etc. To attempt to approximate experimental variation in replicate spectra, fragment intensities were also randomized by up to 30% of their initial values (in retrospect, a very severe degradation). The resulting composite, degraded spectra were then searched against the original spectra. FIGS. 8 and 9 show representative results. In plot **110** of FIG. 8, a portion of the search results is portrayed as a contour plot. The hits can be seen as a very prominent wall **112** running along the diagonal.

Although not readily apparent in FIG. 8, the wall **112** has a thickness that is several units wide in cross sections through the results taken parallel to the "Library Entry Number" axis. This property is more readily apparent in plot **120** of FIG. 9, in which the results vectors of eight non-adjacent test spectra (composite spectra synthetically generated as described above) are displayed as line plots. The results vectors are shown in FIG. 9 as line plots **121** (long-dashed line), **122** (short dashed-dotted line), **123** (short-dashed line), **124** (dotted line), **125** (solid line), **126** (long dashed-dotted line), **127** (dashed double-dotted line) and **128** (boxed line). For clarity, fluctuations in the results vectors close to the "0.0" line (essentially "noise") are not shown in FIG. 9. In the idealized case, each line plot would have zero signal except across a set of ten consecutive library entries, representing the parent spectra composited and degraded to make each respective test spectrum. One can observe, from FIG. 9, that it is, in fact, possible to identify a large number of component spectra in these very highly multiplexed cases. Degradation of the test spectra here was severe and was much greater than the spectrum-to-spectrum variation generally encountered in replicate spectra, yet very high degree of multiplexing is nonetheless tolerated by the decomposition routine. However, a few false positive peaks (e.g., small outlying peaks for results vectors **125, 123** and **126)** and a few physically impermissible negative contributions (e.g., in results vectors **123** and **127)** are indicated, possibly as a result of the severe degradation of the composite test spectra.

The discussion included in this application is intended to serve as a basic description. Although the present invention has been described in accordance with the various embodiments shown and described, one of ordinary skill in the art will readily recognize that there could be variations to the embodiments and those variations would be within the spirit and scope of the present invention. The reader should be aware that the specific discussion may not explicitly describe all embodiments possible; many alternatives are implicit. Accordingly, many modifications may be made by one of ordinary skill in the art without departing from the spirit, scope and essence of the invention. Neither the description nor the terminology is intended to limit the scope of the invention. All patent application disclosures, patent application publications or other publications are hereby explicitly incorporated by reference herein as if fully set forth herein. In any instances in which such incorporated material is in conflict with the present disclosure, the present disclosure shall control.

**Claims**

1.  A method of acquiring and compiling data relating to a plurality of chemical compounds on a mass spectrometer system, comprising:

    (a) generating a multiplexed mass spectrum using the mass spectrometer system, the multiplexed mass spectrum comprising a superposition of a plurality of product-ion mass spectra comprising a plurality of product-ion types, each product-ion mass spectrum corresponding to fragmentation of a respective precursor-ion type formed by ionization of the plurality of chemical compounds, each precursor-ion type having a respective precursor-ion mass-to-charge (*m*/*z*) ratio and each product ion type having a respective product-ion *m*/*z* ratio;
    (b) decomposing the multiplexed product-ion mass spectrum so as to recognize relative abundances of previously-observed product-ion mass spectra within the multiplexed product-ion mass spectrum, the decomposing employing a mass-spectral library having a plurality of entries wherein each entry corresponds to a respective previously-observed product ion mass spectrum, said previous observation being made on said mass spectrometer system;
    (c) recognizing an additional contribution to the multiplexed product-ion mass spectrum that is neither attributable to random variation nor to any previously-observed product-ion spectrum; and
    (d) storing at least one new entry in the mass-spectral library relating to the recognized additional contribution.

2.  A method as recited in claim 1, wherein the step (a) of generating a multiplexed mass spectrum using the mass spectrometer system comprises operating the mass spectrometer system according to a rule that specifies instrument operating parameters required for performing the decomposing (b) employing the mass-spectral library.

3. A method as recited in claim 1, wherein the step (d) of storing at least one new entry in the mass spectral library relating to the recognized additional contribution includes storing instrument operating parameters in said mass spectral library entry.

4. A method as recited in claim 1, wherein the plurality of compounds comprises introducing a plurality of compounds that elutes from a chromatograph at a particular retention time and further comprising:

if there exists a recognized additional contribution to the multiplexed product-ion mass spectrum, constructing a respective profile of detected product-ion intensity versus retention time for each product ion contributing to the recognized additional contribution;
recognizing subsets of the profiles based on correlations between the profiles; and
decomposing the additional contribution to the multiplexed product-ion mass spectrum into multiple contributions to the multiplexed product-ion mass spectrum in accordance with the recognized subsets.

5. A method as recited in claim 1, wherein:

the step (a) comprises fragmenting only precursor-ion types having precursor-ion $m/z$ ratios within a restricted range of $m/z$ ratios; and
the step (b) of decomposing the multiplexed product-ion mass spectrum so as recognize relative abundances of previously-observed product-ion mass spectra comprises employing a segment of the mass spectral library, each entry of the segment of the mass spectral library corresponding to precursor-ion types within the restricted range of $m/z$ ratios.

6. A method as recited in claim 1, wherein the step (d) of storing at least one new entry in the mass-spectral library relating to the recognized additional contribution comprises:

(d1) creating an additional column in a matrix **D** defined by

$$\mathbf{D} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \begin{bmatrix} d_{n,k} \end{bmatrix}_{N \times K}$$

wherein each element $d_{n,k}$
is an observed intensity of a $k^{th}$ product-ion spectrum at an $n^{th}$ $m/z$ bin position, $K$ is a total number of $MS^2$ spectra and $N$ is a total number of defined $m/z$ bin positions; and
(d2) storing, in the mass spectral library, each **D** matrix or a respective matrix derived therefrom.

7. A method as recited in claim 1, wherein the step (d2) comprises, storing in the mass spectral library, a matrix calculated as $\mathbf{D}^T\mathbf{D}$.

8. A method as recited in claim 5, wherein the step (d) of storing at least one new entry in the mass-spectral library relating to the recognized additional contribution comprises:

(d1) creating an additional column in a matrix **D** defined by

$$\mathbf{D} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = \left[ d_{n,k} \right]_{N \times K}$$

wherein each element $d_{n,k}$
is an observed intensity of a $k^{th}$ product-ion spectrum of the segment of the mass spectral library at an $n^{th}$ $m/z$ bin position, $K$ is a total number of product-ion spectra assigned to the library segment and $N$ is a total number of $m/z$ bins defined within the library segment; and
(d2) storing, in the mass spectral library, each **D** matrix or a respective matrix derived therefrom.

9. A method as recited in claim 8, wherein the step (d2) comprises, storing in the mass spectral library, a matrix defined as $\mathbf{D}^T\mathbf{D}$.

10. A method as recited in claim 1, wherein the step (a) of generating a multiplexed mass spectrum using the mass spectrometer system comprises:

(a1) introducing, simultaneously, each of the plurality of compounds to an ion source of the mass spectrometer system;
(a2) producing the plurality of the precursor ion types each of the plurality of compounds using an ion source of the mass spectrometer system;
(a3) simultaneously fragmenting said plurality of precursor-ion types so as to form the plurality of product ion types; and
(a4) mass analyzing the plurality of product ion types so as to generate the multiplexed mass spectrum.

11. A method as recited in claim 10, wherein the step (a2) of producing the plurality of precursor ion types comprises:

generating an initial plurality of precursor-ion types within a first range of $m/z$ ratios using the ion source;
selecting a second range of $m/z$ ratios, said second range being a sub-range of the first range of $m/z$ ratios; and
isolating the plurality of precursor ion types as being those precursor ion types whose $m/z$ ratios are within the second range.

12. A method as recited in claim 1, wherein each entry in the mass spectral library comprises information relating to $m/z$ ratios and detected intensities of previously-observed product ions of the previously-observed product ion mass spectrum.

13. A method of compiling data previously obtained on a mass spectrometer system into a local mass spectral library, comprising:

(a) reading a plurality of tandem mass spectra previously obtained using the mass spectrometer system, each tandem mass spectrum comprising detected intensity data for a precursor ion type having a respective precursor-ion mass-to-charge ($m/z$) ratio and an $MS^2$ spectrum comprising detected intensity data for one or more product ion types formed by fragmentation of the precursor ion type and having respective product-ion $m/z$ ratios;
(b) sorting the tandem mass spectra according to the precursor-ion $m/z$ ratios;
(c) assigning each tandem mass spectrum to one of a plurality of library segments according to its respective precursor-ion $m/z$ ratio, each library segment representing a respective range of precursor-ion $m/z$ ratios;
(d) assigning each of the product ion types within each library segment to one of a plurality of bins defined for the library segment, each bin representing a respective range of product-ion $m/z$ ratios;
(e) computing, for each library segment, a matrix **D** defined by

$$\mathbf{D} = \begin{bmatrix} d_{1,1} & d_{1,2} & \cdots & d_{1,K} \\ d_{2,1} & d_{2,2} & \cdots & d_{2,K} \\ \vdots & \vdots & \ddots & \vdots \\ d_{N,1} & d_{N,2} & \cdots & d_{N,K} \end{bmatrix} = [d_{n,k}]_{N \times K}$$

wherein each element $d_{n,k}$
is the observed intensity of the $k^{th}$ MS$^2$ spectrum at the $n^{th}$ bin position, $K$ is the total number of MS$^2$ spectra assigned to the library segment and $N$ is the total number of $m/z$ bins defined within the library segment; and
(f) storing, in the local mass spectral library, each **D** matrix or a respective matrix derived therefrom.

14. A method as recited in claim 14, further comprising, prior to the storing step (f), the steps of:

(e1) calculating the matrix $\mathbf{D}^T\mathbf{D}$; and
(e2) consolidating redundant entries in the **D** matrix based on correlations observed from the $\mathbf{D}^T\mathbf{D}$ matrix.

15. A method as recited in claim 14, wherein the step (f) comprises storing each $\mathbf{D}^T\mathbf{D}$ matrix in the mass spectral library.

16. A method as recited in claim 14, further comprising, prior to the storing step (f):

(e3) calculating each inverse matrix, $(\mathbf{D}^T\mathbf{D})^{-1}$.

17. A method as recited in claim 14, wherein the step (f) comprises storing each matrix $(\mathbf{D}^T\mathbf{D})^{-1}$ in the mass spectral library.

18. A method as recited in claim 13, further comprising:

identifying MS$^2$ spectra in at least one **D** matrix using a conventional identification method that recognizes the spectra based on $m/z$ ratios but not detected intensities.

19. A method as recited in claim 18, further comprising filtering the entries in the at least one **D** matrix so as to only include identified MS$^2$ spectra.

20. A method as recited in claim 18, further comprising storing, in the local mass spectral library, an annotation relating to at least one identified MS$^2$ spectrum.

**FIG. 1**
**(Prior Art)**

<u>10</u>

**FIG. 2**

FIG. 3A

50   54

52   56

FIG. 3B

61

FIG. 3C

52

62   62   62   62   62

FIG. 3D

54

64   64   64   64

FIG. 3E

56

66   66   66   66   66   66

**FIG. 4**

**FIG. 5**

EP 2 741 224 A1

FIG. 6

EP 2 741 224 A1

FIG. 7

EP 2 741 224 A1

FIG. 8

FIG. 9

Europäisches
Patentamt

European
Patent Office

Office européen
des brevets

**EUROPEAN SEARCH REPORT**

Application Number

EP 13 19 3508

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 410 609 A (AGILENT TECHNOLOGIES INC [US]) 3 August 2005 (2005-08-03) * the whole document * | 1-20 | INV. G06F19/00 |
| X | WO 02/21428 A1 (LARGE SCALE PROTEOMICS CORP [US]; ANDERSON N LEIGH [US]; ANDERSON NORM) 14 March 2002 (2002-03-14) * the whole document * | 1-20 | |
| A | DROMEY R. G.: "Simple index for classifying mass spectra with applications to fast library searching", ANALYTICAL CHEMISTRY, vol. 48, no. 11, 1 September 1976 (1976-09-01), pages 1464-1469, XP055116082, ISSN: 0003-2700, DOI: 10.1021/ac50005a013 * the whole document * | 1-20 | |
| A | KIND T. ET AL: "FiehnLib: Mass Spectral and Retention Index Libraries for Metabolomics Based on Quadrupole and Time-of-Flight Gas Chromatography/Mass Spectrometry", ANALYTICAL CHEMISTRY, vol. 81, no. 24, 23 November 2009 (2009-11-23), pages 10038-10048, XP055116083, ISSN: 0003-2700, DOI: 10.1021/ac9019522 * page 10038, right-hand column, line 22 - page 10039, left-hand column, line 39 * | 4 | TECHNICAL FIELDS SEARCHED (IPC) G06F |

-/--

The present search report has been drawn up for all claims

| Place of search | Date of completion of the search | Examiner |
|---|---|---|
| The Hague | 30 April 2014 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 13 19 3508

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WAGNER C. ET AL: "Construction and application of a mass spectral and retention time index database generated from plant GC/EI-TOF-MS metabolite profiles", PHYTOCHEMISTRY, PERGAMON PRESS, GB, vol. 62, no. 6, 1 March 2003 (2003-03-01), pages 887-900, XP004408975, ISSN: 0031-9422, DOI: 10.1016/S0031-9422(02)00703-3 * the whole document * | 4 | |
| A | HOPLEY CH. ET AL: "Towards a universal product ion mass spectral library - reproducibility of product ion spectra across eleven different mass spectrometers", RAPID COMMUNICATIONS IN MASS SPECTROMETRY, vol. 22, no. 12, 9 May 2008 (2008-05-09), pages 1779-1786, XP055116085, ISSN: 0951-4198, DOI: 10.1002/rcm.3545 * the whole document * | 1-20 | |

| | TECHNICAL FIELDS SEARCHED (IPC) |
|---|---|
| | |

The present search report has been drawn up for all claims

| Place of search | Date of completion of the search | Examiner |
|---|---|---|
| The Hague | 30 April 2014 | Godzina, Przemyslaw |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................................

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                    EP 13 19 3508

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2014

| Patent document cited in search report | | | Publication date | Patent family member(s) | | Publication date |
|---|---|---|---|---|---|---|
| GB 2410609 | A | | 03-08-2005 | GB 2410609 A | | 03-08-2005 |
| | | | | US 2005167582 A1 | | 04-08-2005 |
| WO 0221428 | A1 | | 14-03-2002 | AU 8850101 A | | 22-03-2002 |
| | | | | WO 0221428 A1 | | 14-03-2002 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02078046 A
- US 5886346 A
- US 6872938 B